# EUROPÄISCHE PATENTSCHRIFT

(11) **EP 0 639 175 B1**
(45) Veröffentlichungstag und Bekanntmachung des Hinweises auf die Patenterteilung: **29.05.1996**
(21) Anmeldenummer: 92902002.2
(22) Anmeldetag: 12.12.1991
(51) Int. Cl.: C07C 69/708, C07C 67/31

(54) **VERFAHREN ZUR HERSTELLUNG VON 2-(DIALKOXYMETHYL)-CARBONSÄUREESTERN**
PROCESS FOR PRODUCING 2-(DIALKYLOXY METHYL)-CARBOXYLIC ACID ESTERS
PROCEDE DE FABRICATION D'ESTERS DE L'ACIDE 2-(DIALCOXYMETHYLE)-CARBOXYLIQUE

(30) Priorität: 05.01.1991 DE 4100178
(43) Veröffentlichungstag der Anmeldung: 22.02.1995
(73) Patentinhaber: HOECHST AKTIENGESELLSCHAFT, D-65926 Frankfurt am Main (DE)
(72) Erfinder: STRUTZ, Heinz, D-6230 Frankfurt am Main 80 (DE)
(86) Internationale Anmeldenummer: EP9102388
(87) Internationale Veröffentlichungsnummer: WO9212116

(56) Entgegenhaltungen:
- EP-A- 0 055 108

## Beschreibung

Die Erfindung betrifft ein Verfahren zur Herstellung von 2-(Dialkoxymethyl)-carbonsäureestern der Formel I (siehe Patentanspruch 1).

Verbindungen der Formel I sind gut handhabbare, da stabile Derivate der Formylessigester. Sie werden als Zwischenprodukte für die Herstellung entsprechender Vinyläther sowie bei der Herstellung von Riechstoffen, Pharmazeutika und Pflanzenschutzpräparaten, eingesetzt.

Es ist bekannt, 2-(Dialkoxymethyl)-carbonsäureester durch Reaktion von Orthoameisensäureestern mit Keten in Gegenwart von BF₃ als Katalysator herzustellen. Bei dem beschriebenen Verfahren (US-PS 2 449 471) werden Verbindungen der Formel I mit R = H und R¹ = R³ erhalten. In der Praxis ist die Einsetzbarkeit dieses Verfahrens stark eingeschränkt, da das instabile Keten nur begrenzt lagerfähig ist. Weitere Nachteile dieses Verfahrens sind, daß mit einem hohen Überschuß an Keten gearbeitet und nur eine geringe Ausbeute von ca. 55 % erzielt wird. Weiterhin sind auf diese Weise nur "symmetrische" Produkte mit R¹ = R³ erhältlich.

Nach der US-PS 2 535 012 werden 2-(Dialkoxymethyl)-carbonsäureester durch Reaktion von Acetylen mit Dialkylcarbonaten unter Natriumalkoholat-Katalyse hergestellt. Nachteilig sind hier der anzuwendende hohe Überschuß an Dialkylcarbonat und die nur mäßigen Ausbeuten. Darüberhinaus bestehen erhebliche Probleme bei der destillativen Abtrennung des mitentstehenden β-Alkoxyacrylsäureesters (Houben/Weyl, Bd. VII/I, S. 109).

Weiterhin ist die Addition von Alkoholen an das Natriumsalz des Formylessigesters in Gegenwart von Säure im Überschuß (DE-OS 3641605) oder an β-Alkoxy- bzw. β-Carbalkoxyacrylsäureester (DE-OS 3211679) bekannt, die ihrerseits aus dem Natriumsalz des Formylessigesters oder durch Anlagerung von Alkoholen oder Carbonsäuren an Propiolester hergestellt werden. Der Einsatz des Natriumsalzes des Formylessigesters hat den Nachteil, daß zu seiner Herstellung stöchiometrische Mengen Alkalialkoholat, das aus Alkalimetall gebildet werden muß, benötigt werden. Dadurch, sowie durch die notwendige Neutralisation des Säureüberschusses, fallen unerwünschte Salzmengen an. Die Verwendung von Vinyläthern und -estern als Ausgangsprodukt für 2-(Dialkoxymethyl)-carbonsäureester ist wegen ihrer mehrstufigen und aufwendigen Herstellung unvorteilhaft. Im allgemeinen stellt man Vinyläther sogar umgekehrt aus entsprechenden Acetalen durch Alkoholabspaltung her (EP-OS 0327985). Nach JP-OS 60-156643 lassen sich 2-(Dialkoxymethyl)-carbonsäureester durch säurekatalysierte Alkoholyse von β-Alkoxy-β-carbamidopropionsäureestern herstellen. Diese sind jedoch nur aufwendig durch elektrochemische Oxidation von β-Amidopropionsäureestern zu erhalten, die wiederum erst durch Anlagerung von Aminen an Acrylsäureester in Anwesenheit des entsprechenden Säureanhydrids oder -chlorids erhältlich sind.

Nach der EP-OS 55 108 werden 2-(Dialkoxymethyl)-carbonsäureester durch Oxidation von Acrylsäureestern in Gegenwart von Katalysatoren mit Alkylnitriten in Anwesenheit von Alkohol hergestellt. Die Ausbeuten an dem gewünschten Produkt liegen jedoch unter 10 %; zudem wird ein enormer Alkylnitrit-Überschuß benötigt (ca. 50fach). Oxidiert man mit Luft in Anwesenheit von PdCl₂ und CuCl₂, sinken die Ausbeuten noch weiter auf unter 3 %. Die Oxidation von Acrylestern mit Sauerstoff unter diesen Bedingungen ist in J. Org. Chem. (1969) 34, 3949 sogar als ungeeignet für die Herstellung von 2-(Dialkoxymethyl)carbonsäureestern beschrieben worden.

Es bestand daher die Aufgabe, ein verbessertes Verfahren zur Herstellung von 2-(Dialkoxymethyl)-carbonsäureestern zur Verfügung zu stellen, das die genannten Nachteile nicht aufweist.

Es wurde nun gefunden, daß Acrylsäureester in Anwesenheit von Metallen der Platingruppe und/oder deren Verbindungen und Kupferverbindungen sowie von Alkoholen mittels Sauerstoff und in hohen Ausbeuten in die 2-(Dialkoxymethyl)-carbonsäureester übergeführt werden können, wenn das Katalysatorsystem 1 bis 4 Äquivalente Anionen, bezogen auf die Summe der Metallatome und Metallkationen, enthält, wovon höchstens 3 Äquivalente Halogenidionen darstellen. Der Sauerstoff kann in reiner Form oder in Form von Gemischen mit inerten Gasen eingesetzt werden, wobei von den Gemischen vorzugsweise Luft eingesetzt wird.

Gegenstand der Erfindung ist nun ein Verfahren zur Herstellung von 2-(Dialkoxymethyl)-carbonsäureestern der Formel I (siehe Patentanspruch 1), worin
- R¹: ein nicht-aromatischer Kohlenwasserstoffrest oder ein heterocyclischer nicht-aromatischer Rest, der außer O-, N- und/oder S-Atomen im Ring nur noch C- und H-Atome enthält, mit jeweils 1 bis 20 C-Atomen ist, wobei der Rest R¹ 1 bis 5 Substituenten tragen kann und die Substituenten gleich oder verschieden sind und Halogen, eine Aryl-, Carbalkoxy-, Dialkylamino-, Diarylamino-, Cyanogruppe oder eine Alkoxygruppe darstellen, wobei das Alkyl substituiertes oder unsubstituiertes C₁- bis C₁₂-Alkyl ist, oder eine Aryloxygruppe, wobei das Aryl substituiertes oder unsubstituiertes C₆- bis C₁₄-Aryl ist, einen substituierten oder unsubstituierten Benzyloder Phenäthylrest oder eine R⁴O-(R⁵O)ₓ-R⁵-Gruppe darstellt, wobei R⁴ substituiertes oder unsubstituiertes C₁- bis C₁₂-Alkyl oder C₆- bis C₁₄-Aryl ist, R⁵ ein verzweigter oder unverzweigter Kohlenwasserstoffrest mit 1 bis 4 C-Atomen oder Phenylen und x = 1 bis 6 ist,
- R: Wasserstoff oder Alkyl mit 1 bis 5 C-Atomen und
- R³: unsubstituiertes oder substituiertes C₆- bis C₁₄-Aryl oder ein unter R¹ genannter Rest ist,
durch oxidierende β-Acetalisierung von Acrylsäureestern mit Alkoholen R¹OH, worin R¹ die genannte Bedeutung hat, unter Einwirkung von Sauerstoff als Oxidationsmittel und eines Katalysatorsystems auf der Basis von einem oder mehreren Metallen der Platingruppe und/oder deren Verbindungen und einer Kupferverbindung, das dadurch gekennzeichnet ist, daß die Reaktion bei einer Temperatur von bis zu 80°C durchgeführt wird und das Katalysatorsystem 1 bis 4 Äquivalente Anionen, bezogen auf die Summe der Metallatome und Metallkationen, enthält, wovon höchstens 3 Äquivalente Halogenidionen sind, wobei das Halogen ein Molekulargewicht von mindestens 35 aufweist.

Der Begriff Acrylsäureester umfaßt in diesem Zusammenhang auch Acrylsäureester, die in α-Position substituiert sind. Bevorzugt sind jedoch unsubstituierte Acrylsäureester.

Der Rest R³ hat keinen wesentlichen Einfluß auf die Reaktion und kann daher im allgemeinen beliebig gewählt werden, sofern er unter den Reaktionsbedingungen inert ist.

Die alkoholische Reaktionskomponente ist in weitem Rahmen frei wählbar und kann an einer oder mehreren Positionen durch solche funktionellen Gruppen substituiert sein, die unter den oxidierenden Reaktionsbedingungen oder bzgl. der eigenen Reaktion mit dem in der Reaktion auf die formale Oxidationsstufe +1 aufoxidierten β-Kohlenstoffatom inert sind.

Als Beispiele für den Alkohol seien genannt Methanol, Äthanol, n- und i-Propanol, die verschiedenen Butanole, Cyclohexanol, gegebenenfalls substituierter Benzylalkohol, gegebenenfalls substituierter Phenäthylalkohol, 2-Chloräthanol, 3-Chlorpropanol, Monomethyl-, -äthyl-, -propyl, -butyl- oder -phenyläther von Glykolen und anderen zweiwertigen Alkoholen oder von Polymerisationsprodukten derartiger Alkohole, 2-Dimethylaminoäthanol, N-(2-Hydroxyäthyl)formamid, 3-Hydroxypropionsäurenitril, Glykolsäureester und 3,3-Dimethoxy-propanol-1; besonders bevorzugt sind aliphatische, in 1-Position nicht verzweigte Alkohole, wie Methanol, Äthanol, n-Propanol, n-Butanol sowie das 2-Methylpropanol. Es können aber auch Gemische von Alkoholen eingesetzt werden. Unter Glykol ist hier insbesondere Äthylenglykol, Propandiol-1,2 und Butandiol-1,2 zu verstehen. Als andere zweiwertige Alkohole seien z. B. Propandiol-1,3 und Butandiol-1,4 genannt.

Der Alkohol kann, sofern er als Flüssigkeit bei Reaktionstemperatur vorliegt, auch als Lösungsmittel dienen. Wenn ein anderes Lösungsmittel angewendet wird, wird der Alkohol bevorzugt in der mindestens zweifachen Molmenge, besonders bevorzugt in der mindestens achtfachen Molmenge, bezogen auf den Acrylsäureester eingesetzt. Die Menge des zugesetzten Lösungsmittels wird zweckmäßig so bemessen, daß das Reaktionsgemisch gut handhabbar ist. Als Lösungsmittel können insbesondere aprotische polare Verbindungen eingesetzt werden, die mit den Reaktionsteilnehmern mischbar sind bzw. diese lösen, ohne mit diesen zu reagieren, und die den Katalysator zumindest anlösen. Eine Reaktion mit dem Katalysator oder Co-Katalysator im Sinne einer Koordination braucht nicht ausgeschlossen zu sein. Beispiele für solche Lösungsmittel sind Diäther der obengenannten Glykole und anderer zweiwertiger Alkohole oder aliphatische oder aromatische Nitrile. Besonders bevorzugt sind Dimethoxyäthan, Acetonitril und Benzonitril.

Die Komponenten des Katalysatorsystems werden zweckmäßig als solche oder auf einem geeigneten Träger, wie Aluminiumoxid, Silicagel oder Kohlenstoff, fixiert eingesetzt. Im letzteren Fall kann das Verfahren kontinuierlich durchgeführt werden. Aus der Gruppe der Platinmetalle sind Palladium und seine Verbindungen bevorzugt. Besonders bevorzugt sind Palladiumhalogenide PdX₂ oder Palladium-Nitril-Komplexe PdX₂(NCR)₂ , worin das Halogen X ein Molekulkargewicht von mindestens 35 aufweist und R ein Kohlenwasserstoffrest mit 1 bis 8 C-Atomen, bevorzugt Phenyl, ist. Als weitere Verbindungen seien beispielsweise genannt Pd(CH₃COO)₂ und Palladium-bis-acetylacetonat. Das Metall der Platingruppe und/oder seine Verbindungen können vorteilhaft in einem Mol-Verhältnis von 10⁻⁵ bis 10, vorzugsweise 10⁻⁴ bis 0,2, besonders bevorzugt 10⁻³ bis 0,1, bezogen auf den Acrylsäureester, eingesetzt werden, wobei vorzugsweise das Atomverhältnis des Kupfers zum Metall der Platingruppe mindestens 1:1 ist.

Als Co-Katalysatoren werden Verbindungen des Kupfers in der Oxidationsstufe +1, die vorteilhaft wenig oder kein Wasser enthalten, eingesetzt. Bevorzugt sind Halogenide und Pseudohalogenide des Kupfers in der Oxidationsstufe +1. Besonders bevorzugt sind CuCl, CuBr und CuJ. Die Kupferverbindung wird vorteilhaft in einem Mol-Verhältnis von 10⁻⁵ bis 10, bevorzugt 10⁻³ bis 5, besonders bevorzugt 0,01 bis 1, bezogen auf den Acrylsäureester, eingesetzt. Ein eventuell ungelöst im Reaktionsmedium vorliegende Anteil der Kupferverbindung kann nach der Reaktion zurückgewonnen werden. Das Katalysatorsystem kann bei entsprechender Reaktionsführung mehrfach eingesetzt werden.

Der Sauerstoff dient als Oxidationsmittel zur Überführung des β-Kohlenstoffatoms der Acrylsäureester von der Oxidationsstufe -2 zur Oxidationsstufe +1. Die Zufuhr des Oxidationsmittels kann beispielsweise durch Über- oder Durchleiten erfolgen. Die Oxidation kann im Rahmen der geltenden Sicherheitsbestimmungen für den Umgang mit Luft-bzw. Sauerstoff-Kohlenwasserstoffgemischen bei Atmosphärendruck oder Überdruck durchgeführt werden.

Die Reaktionstemperatur liegt im allgemeinen oberhalb -10°C, bevorzugt zwischen +10 und +70°C, besonders bevorzugt zwischen +20 und +55°C. Das Überschreiten einer Reaktionstemperatur von ca. 70°C kann sich in Abhängigkeit von den übrigen Reaktionsbedingungen ungünstig auf die Ausbeute an 2-(Dialkoxymethyl)carbonsäureester auswirken.

Die Reaktionszeiten sind von den sonstigen Reaktionsparametern wie Temperatur, Druck und Katalysatorkonzentration abhängig und liegen im allgemeinen im Rahmen von z.B. 5 bis 50 Stunden, meistens 10 bis 30 Stunden.

Die Aufarbeitung erfolgt nach üblichen Methoden. Sie ist unproblematisch, da sich die Reaktion leicht so lenken läßt, daß das Produkt praktisch frei von den entsprechenden 3-Alkoxyacrylsäureestern ist. Eine Neutralisierung des Reaktionsgemisches ist nicht notwendig, so daß im Gegensatz zum Stand der Technik bei der Aufarbeitung kein Salz anfällt.

Ein weiterer Vorteil des erfindungsgemäßen Verfahrens ist, daß 2-(Dialkoxymethyl)-carbonsäureester der allgemeinen Formel I in einem einzigen Schritt aus leicht zugänglichen, technisch problemlos handhabbaren Ausgangsverbindungen bei optimaler Reaktionsführung in hohen Ausbeuten von mehr als 80 %, bezogen auf den eingesetzten Acrylsäureester hergestellt werden können. Dieses Ergebnis ist insbesondere deshalb überraschend, weil die Herstellung von 2-(Dialkoxymethyl)-carbonsäureestern aus Acrylsäure-Derivaten durch Oxidation mit Luft in Anwesenheit von PdCl₂ und CuCl₂, wie eingangs erwähnt, bisher nur zu extrem schlechten Ausbeuten geführt hat.

### Beispiele

1. In einem Rundkolben mit Magnetkern und Rückflußkühler wurden 0,21 g (1,2 mmol) PdCl₂, 1,2 g (12 mmol) CuCl, 3,42 g (108 mmol) Methanol und 1,53 g (12 mmol) Acrylsäurebutylester in 10,5 g Dimethoxyäthan 20 Stunden bei 50°C unter O₂-Atmosphäre (O₂-gefüllter Luftballon) gerührt. Nach Zusatz von 1,2 g m-Phenoxytoluol als innerem Standard wurden gaschromatographisch 1,57 g (8,2 mmol) 3,3-Dimethoxypropionsäurebutylester (69 % d.Th.) gefunden. Nach Filtration über basisches Al₂O₃ (Aktivität I; Hexan/Methyl-t-butyläther (1:1) als Eluenz) wurden bei 45°C/0,1 bar die flüchtigen Bestandteile abgezogen, und im Rückstand ¹H-NMR-spektroskopisch 1,62 g (8,5 mmol) 3,3-Dimethoxypropionsäurebutylester (71 % d.Th.) in sehr guter Übereinstimmung mit der gaschromatographischen Analyse gefunden.
2. bis 8. In Analogie zum Beispiel 1 wurden die Einflüsse der CuCl-Konzentration, der Reaktionstemperatur und der Reaktionszeit untersucht. Die Ergebnisse sind in Tabelle 1 zusammengefaßt.

**Tabelle 1**

| Bsp. | Masse (CuCl) (g) | T(°C) | t(h) | Ausbeute (%*) |
|---|---|---|---|---|
| 2 | 0,6 | 50 | 20 | 76 |
| 3 | 0,3 | 50 | 20 | 67 |
| 4 | 0,6 | 40 | 20 | 81 |
| 5 | 0,3 | 40 | 20 | 86 |
| 6 | 0,3 | 30 | 20 | 82 |
| 7 | 0,15 | 40 | 30 | 78 |
| 8 | 0,6 | 70 | 20 | 28 |

| | | | | |
|---|---|---|---|---|
| *gaschromatographisch bestimmt; m-Phenoxytoluol (1,2 g) als innerer Standard | | | | |

9. und 10. In einem Rundkolben mit Magnetkern und Rückflußkühler wurden 0,07 g (0,4 mmol) PdCl₂, 4 mmol einer Cu-Verbindung entsprechend Tabelle 2, 1,14 g (36 mmol) Methanol und 0,51 g Acrylsäurebutylester in 3,5 g Dimethoxyäthan 20 Stunden bei 50°C unter O₂-Atmosphäre (O₂-gefüllter Luftballon) gerührt. Die Ausbeuten an 3,3-Dimethoxypropionsäurebutylester in Abhängigkeit von der verwendeten Cu-Verbindung sind in Tabelle 2 zusammengestellt.

**Tabelle 2**

| Bsp. | Cu-Verbindung | Ausbeute (%*) |
|---|---|---|
| 9 | Cu(OAc)₂ | 30 |
| 10 | CuSO₄ | 30 |

| | | |
|---|---|---|
| *gaschromatographisch bestimmt; m-Phenoxytoluol (0,4 g) als innerer Standard | | |

11. Beispiel 2 wurde wiederholt, aber anstelle von PdCl₂ wurden 0,27 g (1,2 mmol) Pd(OAc)₂ eingesetzt. Gaschromatographisch wurden 1,29 g (6,8 mmol) 3,3-Dimethoxypropionsäurebutylester (57 % d.Th.) gefunden.
12. Beispiel 2 wurde wiederholt, aber anstelle von PdCl₂ wurden 0,12 g (0,3 mmol) PdCl₂(NCPh)₂ eingesetzt. Gaschromatographisch wurden 1,96 g (10,3 mmol) 3,3-Dimethoxypropionsäurebutylester (86 % d.Th.) gefunden. Nach Aufarbeitung entsprechend Beispiel 1 findet man ¹H-NMR-spektroskopisch 2,0 g (10,5 mmol) des Produkts (88 % d.Th.) in sehr guter Übereinstimmung mit der gaschromatographischen Analyse.
13. Beispiel 12 wurde wiederholt, aber anstelle von Methanol wurden 5 g (109 mmol) Äthanol eingesetzt. Gaschromatographisch werden 2,25 g (9,8 mmol) 3,3-Diäthoxypropionsäurebutylester (82 % d.Th.) nachgewiesen.
14. Beispiel 12 wurde wiederholt, aber anstelle von Methanol wurden 10,2 g (108 mmol) 3-Chlorpropanol eingesetzt. Gaschromatographisch werden 1,23 g (3,9 mmol) 3,3-Di-(3'-chlorpropoxy)-propionsäurebutylester (33 % d.Th.) nachgewiesen.
15. Beispiel 12 wurde wiederholt, aber anstelle von Acrylsäurebutylester wurden 1,03 g (12 mmol) Methylacrylat eingesetzt. Gaschromatographisch wurden 1,52 g (10,3 mmol) 3,3-Dimethoxypropionsäuremethylester (85 % d.Th.) gefunden.
16. bis 18. In einem Rundkolben mit Magnetkern und Rückflußkühler wurden 0,07 g (0,4 mmol) PdCl₂, 0,4 g (4 mmol) CuCl, Methanol in Mengen entsprechend Tabelle 3 und 0,51 g (4 mmol) Butylacrylat 20 Stunden bei 50°C unter O₂-Atmosphäre (O₂-gefüllter Luftballon) gerührt. Die Ausbeuten an 3,3-Dimethoxypropionsäurebutylester in Abhängigkeit von der Methanol-Menge sind in Tabelle 3 zusammengefaßt.

**Tabelle 3**

| Bsp. | Masse (CH₃OH) (g) | Ausbeute (%*) |
|---|---|---|
| 16 | 0,38 | 50 |
| 17 | 0,76 | 66 |
| 18 | 1,14 | 78 |

| | | |
|---|---|---|
| *gaschromatographisch bestimmt; m-Phenoxytoluol (0,4 g) als innerer Standard | | |

19. In einem Rundkolben mit Magnetkern und Rückflußkühler wurden 0,12 g (0,3 mmol) PdCl₂(NCPh)₂, 1,2 g (12 mmol) CuCl, 10,26 g (321 mmol) Methanol und 3,06 g (24 mmol) Butylacrylat in 21 g Dimethoxyäthan 20 Stunden bei 50°C unter O₂-Atmosphäre (O₂-gefüllter Luftballon) gerührt. Nach Zusatz von 2,4 g m-Phenoxytoluol wurde entsprechend Beispiel 1 aufgearbeitet. Es werden 3,84 g (20,2 mmol) 3,3-Dimethoxypropionsäurebutylester (84 % d.Th.) ¹H-NMR-spektroskopisch gefunden.
20. 0,12 g (0,3 mmol) PdCl₂(NCPh)₂, 1,8 g (18 mmol) CuCl, 4,6 g (36 mmol) Butylacrylat und 15,4 g (481 mmol) Methanol wurden in 31,5 g Dimethoxyäthan 20 Stunden bei 50°C unter O₂-Atmosphäre gerührt. Nach Zusatz von 3,6 g m-Phenoxytoluol wurde wie in Beispiel 1 aufgearbeitet. Im Rückstand werden 1H-NMR-spektroskopisch 5,74 g (30,2 mmol) des Produktes (84 % d.Th.) gefunden.
21. Es wurde zunächst wie in Beispiel 19 verfahren. Nach 20 Stunden wurden weitere 3,06 g (24 mmol) Butylacrylat in 10,3 g (322 mmol) Methanol und 21 g Dimethoxyäthan zugesetzt und weitere 20 Stunden bei 50°C unter O₂-Atmosphäre gerührt. Nach Zugabe von 4,8 g m-Phenoxytoluol wurde wie in Beispiel 1 aufgearbeitet. ¹H-NMR-spektroskopisch wurden 7,56 g (39,7 mmol) 3,3-Dimethoxypropionsäurebutylester (83 % d.Th.) gefunden.
22. Beispiel 21 wurde wiederholt, aber nach insgesamt 40 Stunden wurden weitere 3,06 g (24 mmol) Butylacrylat in 10,3 g Methanol und 21 g Dimethoxyäthan zugesetzt. Nach 20 Stunden Reaktionszeit wurden 7,2 g m-Phenoxytoluol zugegeben und entsprechend Beispiel 1 aufgearbeitet. Die Ausbeute an 3,3-Dimethoxypropionsäurebutylester beträgt 11,4 g (59,9 mmol) (84 % d.Th.)
23. Beispiel 12 wurde wiederholt, aber mit Acetonitril als Lösungsmittel. Nach Aufarbeitung entsprechend Beispiel 1 wurden ¹H-NMR-spektroskopisch 1,96 g (10,3 mmol) 3,3-Dimethoxypropionsäurebutylester (86 % d.Th.) gefunden.
24. Beispiel 1 wurde wiederholt, aber anstelle von CuCl wurden 1,72 g (12 mmol) CuBr eingesetzt. Nach Aufarbeitung entsprechend Beispiel 1 wurden 1,64 g (8,6 mmol) 3,3-Dimethoxypropionsäurebutylester (72 % d.Th.) ¹H-NMR-spektroskopisch gefunden.
25. Beispiel 1 wurde wiederholt, aber anstelle von PdCl₂ wurden 0,32 g (1,2 mmol) PdBr₂ eingesetzt. Nach Aufarbeitung entsprechend Beispiel 1 wurden 1,59 g (8,4 mmol) 3,3-Dimethoxypropionsäurebutylester (70 % d.Th.) ¹H-NMR-spektroskopisch gefunden.

## Patentansprüche

1. Verfahren zur Herstellung von 2-(Dialkoxymethyl)-carbonsäureestern der Formel I 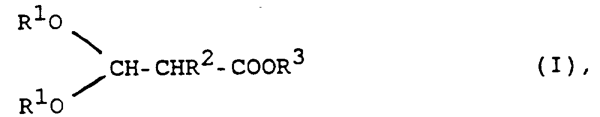 worin
R¹ ein nicht-aromatischer Kohlenwasserstoffrest oder ein heterocyclischer nicht-aromatischer Rest, der außer O-, N- und/oder S-Atomen im Ring nur noch C- und H-Atome enthält, mit jeweils 1 bis 20 C-Atomen ist, wobei der Rest R¹ 1 bis 5 Substituenten tragen kann und die Substituenten gleich oder verschieden sind und Halogen, eine Aryl-, Carbalkoxy-, Dialkylamino-, Diarylamino-, Cyanogruppe oder eine Alkoxygruppe darstellen, wobei das Alkyl substituiertes oder unsubstituiertes C₁- bis C₁₂-Alkyl ist, oder eine Aryloxygruppe, wobei das Aryl substituiertes oder unsubstituiertes C₆- bis C₁₄-Aryl ist, einen substituierten oder unsubstituierten Benzyloder Phenäthylrest oder eine R⁴O-(R⁵O)_{X}-R⁵-Gruppe darstellt, wobei R⁴ substituiertes oder unsubstituiertes C₁- bis C₁₂-Alkyl oder C₆- bis C₁₄-Aryl ist, R⁵ ein verzweigter oder unverzweigter Kohlenwasserstoffrest mit 1 bis 4 C-Atomen oder Phenylen und x = 1 bis 6 ist,
R Wasserstoff oder Alkyl mit 1 bis 5 C-Atomen und
R³ unsubstituiertes oder substituiertes C₆- bis C₁₄-Aryl oder ein unter R¹ genannter Rest ist,
durch oxidierende β-Acetalisierung von Acrylsäureestern mit Alkoholen R¹OH, worin R¹ die genannte Bedeutung hat in Anwesenheit von Sauerstoff als Oxidationsmittel und eines Katalysatorsystems auf der Basis von einem oder mehreren Metallen der Platingruppe und/oder deren Verbindungen und einer Kupferverbindung, dadurch gekennzeichnet, daß die Reaktion bei einer Temperatur von bis zu 80°C durchgeführt wird und das Katalysatorsystem 1 bis 4 Äquivalente Anionen, bezogen auf die Summe der Metallatome und Metallkationen, enthält, wovon höchstens 3 Äquivalente Halogenidionen sind, wobei das Halogen ein Molekulargewicht von mindestens 35 aufweist.

2. Verfahren nach Anspruch 1, dadurch gekennzeichznet, daß R Wasserstoff ist.

3. Verfahren nach Anspruch 1 oder 2, dadurch gekennzeichnet, daß die Reaktion in aliphatischen oder aromatischen Nitrilen oder Diäther von Glykolen und anderen zweiwertigen Alkoholen oder von Polymerisationsprodukten derartiger Alkohole als Lösungsmittel durchgeführt wird.

4. Verfahren nach Anspruch 3, dadurch gekennzeichnet, daß das Lösungsmittel Dimethoxyäthan, Acetonitril oder Benzonitril ist.

5. Verfahren nach einem oder mehreren der Ansprüche 1 bis 4, dadurch gekennzeichnet, daß als Metall der Platingruppe und/oder deren Verbindungen Palladium und/oder seine Verbindungen eingesetzt werden.

6. Verfahren nach Anspruch 5, dadurch gekennzeichnet, daß als Palladium-Verbindung ein Palladiumhalogenid PdX₂ oder ein Palladium-Nitril-Komplex PdX₂(NCR)₂, worin das Halogen ein Molekulargewicht von mindestens 35 aufweist und R ein Kohlenwasserstoffrest mit 1 bis 8 C-Atomen ist, eingesetzt wird.

7. Verfahren nach einem oder mehreren der Ansprüche 1 bis 6, dadurch gekennzeichnet, daß als Kupferverbindung ein Kupfer-I-halogenid CuX, worin das Halogen ein Molekulargewicht von mindestens 35 aufweist, eingesetzt wird.

8. Verfahren nach einem oder mehreren der Ansprüche 1 bis 7, dadurch gekennzeichnet, daß der Alkohol in einer mindestens zweifachen Molmenge, bevorzugt in einer mindestens achtfachen Molmenge, bezogen auf den Acrylsäureester, eingesetzt wird.

9. Verfahren nach einem oder mehreren der Ansprüche 1 bis 8, dadurch gekennzeichnet, daß das Metall der Platingruppe und/oder seine Verbindungen in einem Molverhältnis von 10⁻⁵ bis 10, vorzugsweise 10⁻⁴ bis 0,2, besonders bevorzugt 10⁻³ bis 0,1, bezogen auf den Acrylsäureester, eingesetzt wird, wobei das Atomverhältnis Cu zum Metall der Platingruppe vorzugsweise mindestens 1:1 ist.

10. Verfahren nach einem oder mehreren der Ansprüche 1 bis 9, dadurch gekennzeichnet, daß die Kupfer-I-Verbindung in einem Molverhältnis von 10⁻⁵ bis 10, vorzugsweise 10⁻³ bis 5, besonders bevorzugt 0,01 bis 1, bezogen auf den Acrylsäureester, eingesetzt wird.

11. Verfahren nach einem oder mehreren der Ansprüche 1 bis 10, dadurch gekennzeichnet, daß die Reaktion bei Temperaturen von oberhalb -10°C, vorzugsweise bei +10 bis +70°C, besonders bevorzugt bei +20 bis +55°C, durchgeführt wird.

## Claims

1. A process for the preparation of 2-(dialkoxymethyl)-carboxylic acid esters of the formula I 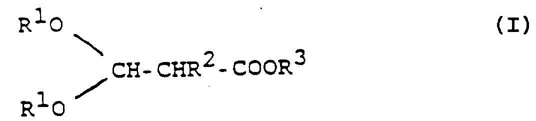 wherein
R¹ is a non-aromatic hydrocarbon radical or a heterocyclic non-aromatic radical, which apart from oxygen, nitrogen and/or sulfur atoms in the ring only contains carbon and hydrogen atoms, in each case having 1 to 20 carbon atoms, it being possible for the radical R¹ to carry 1 to 5 substituents and the substituents being identical or different and being halogen, an aryl, carbalkoxy, dialkylamino, diarylamino or cyano group or an alkoxy group, the alkyl being substituted or unsubstituted C₁- to C₁₂-alkyl, or is an aryloxy group, the aryl being substituted or unsubstituted C₆- to C₁₄-aryl, a substituted or unsubstituted benzyl or phenethyl radical or an R⁴O-(R⁵C)_{X}-R⁵ group, where R⁴ is substituted or unsubstituted C₁- to C₁₂-alkyl or C₆- to C₁₄-aryl, R⁵ is a branched or unbranched hydrocarbon radical having 1 to 4 carbon atoms or phenylene and x = 1 to 6,
R is hydrogen or alkyl having 1 to 5 carbon atoms and
R³ is unsubstituted or substituted C₆- to C₁₄-aryl or a radical mentioned under R¹,
by oxidizing β-acetalization of acrylic acid esters with alcohols R¹OH, wherein R¹ has the meaning mentioned, in the presence of oxygen as the oxidant and of a catalyst system based on one or more metals of the platinum group and/or compounds thereof and on a copper compound, which comprises carrying out the reaction at a temperature of up to 80°C and the catalyst system containing 1 to 4 equivalents of anions, based on the sum of the metal atoms and metal cations, of which at most 3 equivalents are halide ions, the halogen having a molecular weight of at least 35.

2. The process as claimed in claim 1, wherein R is hydrogen.

3. The process as claimed in claim 1 or 2, wherein the reaction is carried out in aliphatic or aromatic nitriles or diethers of glycols and other dihydric alcohols or of polymerization products of alcohols of this type as solvents.

4. The process as claimed in claim 3, wherein the solvent is dimethoxyethane, acetonitrile or benzonitrile.

5. The process as claimed in one or more of claims 1 to 4, wherein palladium and/or its compounds are employed as the metal of the platinum group and/or its compounds.

6. The process as claimed in claim 5, wherein a palladium halide PdX₂ or a palladium/nitrile complex PdX₂ (NCR)₂, in which the halogen has a molecular weight of at least 35 and R is a hydrocarbon radical having 1 to 8 carbon atoms, is employed as the palladium compound.

7. The process as claimed in one or more of claims 1 to 6, wherein a copper(I) halide CuX, in which the halogen has a molecular weight of at least 35, is employed as the copper compound.

8. The process as claimed in one or more of claims 1 to 7, wherein the alcohol is employed in an at least two-fold molar amount, preferably in an at least eight-fold molar amount, relative to the acrylic acid ester.

9. The process as claimed in one or more of claims 1 to 8, wherein the metal of the platinum group and/or its compounds is employed in a molar ratio of 10⁻⁵ to 10, preferably 10⁻⁴ to 0.2, particularly preferably 10⁻³ to 0.1, relative to the acrylic acid ester, the atomic ratio Cu to the metal of the platinum group preferably being at least 1:1.

10. The process as claimed in one or more of claims 1 to 9, wherein the copper(I) compound is employed in a molar ratio of 10⁻⁵ to 10, preferably 10⁻³ to 5, particularly preferably 0.01 to 1, relative to the acrylic acid ester.

11. The process as claimed in one or more of claims 1 to 10, wherein the reaction is carried out at temperatures of above -10°C, preferably at +10 to +70°C, particularly preferably at +20 to +55°C.

## Revendications

1. Procédé de préparation d'esters d'acides 2-(dialcoxyméthyl)-carboxyliques de formule I 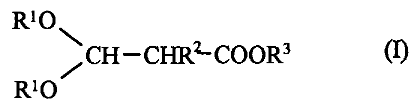 dans laquelle
R¹ représente un reste hydrocarboné non aromatique ou un reste hétérocyclique non aromatique qui, en plus d'atomes d'oxygène, d'azote et/ou de soufre, ne contient dans le noyau que des atomes de carbone et d'hydrogène, de 1 à 20 atomes de carbone, le reste R¹ pouvant porter 1 à 5 substituants qui sont identiques ou différents et qui représentent des halogènes, des groupes aryle, carbalcoxy, dialkylamino, diarylamino, cyano, ou des groupes alcoxy dans lesquels l'alkyle est un alkyle en C₁-C₁₂ substitué ou non substitué, ou des groupes aryloxy dans lesquels l'aryle est un aryle en C₆-C₁₄ substitué ou non substitué, des restes benzyle ou phénéthyle substitués ou non substitués, ou des groupes R⁴O-(R⁵O)_{X}-R⁵-, dans lesquels R⁴ est un reste alkyle en C₁-C₁₂ ou aryle en C₆-C₁₄ substitué ou non substitué, R⁵ est un reste hydrocarboné linéaire ou ramifié de 1 à 4 atomes de carbone ou phénylène et x = 1 à 6,
R est un atome d'hydrogène ou un reste alkyle de 1 à 5 atomes de carbone, et
R³ est un reste aryle en C₆-C₁₄ non substitué ou substitué ou l'un des restes cités pour R¹,
par β-acétalisation dans des conditions oxydantes d'esters d'acides acryliques avec des alcools R¹OH où R¹ a la signification indiquée, en présence d'oxygène comme agent d'oxydation et d'un système catalyseur à base d'un ou plusieurs métaux du groupe du platine et/ou de leurs composés et d'un composé du cuivre, caractérisé en ce que la réaction s'effectue à une température allant jusqu'à 80°C et en ce que le système catalyseur contient, par rapport à la somme des atomes métalliques et des cations métalliques, 1 à 4 équivalents d'anions, dont au plus 3 équivalents sont des ions halogénures, l'halogène ayant une masse moléculaire d'au moins 35.

2. Procédé selon la revendication 1, caractérisé en ce que R est un atome d'hydrogène.

3. Procédé selon la revendication 1 ou 2, caractérisé en ce que la réaction s'effectue dans des nitriles aliphatiques ou aromatiques ou dans des diéthers de glycols et d'autres dialcools ou de produits de polymérisation de ces alcools, en tant que solvants.

4. Procédé selon la revendication 3, caractérisé en ce que le solvant est le diméthoxyéthane, l'acétonitrile ou le benzonitrile.

5. Procédé selon l'une ou plusieurs des revendications 1 à 4, caractérisé en ce que l'on utilise comme métal du groupe du platine et/ou ses composés du palladium et/ou ses composés.

6. Procédé selon la revendication 5, caractérisé en ce que l'on utilise comme composés du palladium un halogénure de palladium PdX₂ ou un complexe de palladium-nitrile PdX₂(NCR)₂, où l'halogène X a une masse moléculaire d'au moins 35 et R est un reste hydrocarboné de 1 à 8 atomes de carbone.

7. Procédé selon l'une ou plusieurs des revendications 1 à 6, caractérisé en ce que l'on utilise comme composé du cuivre un halogénure de cuivre-I CuX, l'halogène ayant une masse moléculaire d'au moins 35.

8. Procédé selon l'une ou plusieurs des revendications 1 à 7, caractérisé en ce que l'on utilise l'alcool en une quantité au moins deux fois molaire, de préférence en une quantité au moins huit fois molaire par rapport à l'ester d'acide acrylique.

9. Procédé selon l'une ou plusieurs des revendications 1 à 8, caractérisé en ce que l'on utilise le métal du groupe du platine et/ou ses composés en un rapport molaire de 10⁻⁵ à 10, de préférence de 10⁻⁴ à 0,2, de façon particulièrement préférée de 10⁻³ à 0,1, par rapport à l'ester d'acide acrylique, le rapport molaire du cuivre au métal du groupe du platine étant de préférence d'au moins 1:1.

10. Procédé selon l'une ou plusieurs des revendications 1 à 9, caractérisé en ce que l'on utilise le composé de cuivre-I en un rapport molaire de 10⁻⁵ à 10, de préférence de 10⁻³ à 5, de façon particulièrement préférée de 0,01 à 1, par rapport à l'ester d'acide acrylique.

11. Procédé selon l'une ou plusieurs des revendications 1 à 10, caractérisé en ce que la réaction s'effectue à des températures supérieures à -10°C, de préférence comprises entre +10 et +70°C, de façon particulièrement préférée entre +20 et +55°C.
